(19) 

Europäisches
Patentamt
European
Patent Office
Office européen
des brevets

(11) **EP 2 039 673 A1**

(12) **EUROPEAN PATENT APPLICATION**
published in accordance with Art. 153(4) EPC

(43) Date of publication:
**25.03.2009 Bulletin 2009/13**

(21) Application number: **07743542.8**

(22) Date of filing: **17.05.2007**

(51) Int Cl.:
*C07C 51/41* $^{(2006.01)}$      *C07C 53/10* $^{(2006.01)}$
*C07C 55/06* $^{(2006.01)}$      *C07C 55/08* $^{(2006.01)}$
*C07C 55/24* $^{(2006.01)}$      *C07C 59/06* $^{(2006.01)}$
*C07C 59/08* $^{(2006.01)}$      *C07C 59/105* $^{(2006.01)}$
*C07C 59/245* $^{(2006.01)}$     *C07C 59/255* $^{(2006.01)}$
*C07C 59/265* $^{(2006.01)}$

(86) International application number:
**PCT/JP2007/060107**

(87) International publication number:
**WO 2008/007497 (17.01.2008 Gazette 2008/03)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **10.07.2006 JP 2006189817**

(71) Applicant: **Nippon Chemical Industrial Co., Ltd.
Koto-ku, Tokyo 136-8515 (JP)**

(72) Inventors:
• **BANDA, Tomohiro
Shunan-shi, Yamaguchi 745-0024 (JP)**
• **KOIKE, Shogo
Shunan-shi, Yamaguchi 745-0024 (JP)**
• **HARA, Takashi
Shunan-shi, Yamaguchi 745-0024 (JP)**

(74) Representative: **Jenkins, Peter David
Page White & Farrer
Bedford House
John Street
London WC1N 2BF (GB)**

(54) **AQUEOUS SOLUTIONS OF ORGANIC ACID CHROMIUM(III) SALTS AND PROCESS FOR PREPARATION THEREOF**

(57) An aqueous solution containing an organic acid chromium (III) salt represented by general formula: $Cr_m(A^x)_n$, wherein A represents a residue left after proton removal from an organic acid; x represents a charge of A; and m and n represent integers satisfying equation $3m+xn=0$, is disclosed. The aqueous solution contains the organic acid chromium (III) salt in a concentration of 6% by weight or higher in terms of $Cr_m(A^x)_n$, has impurity ion concentrations of Na$\leq$30 ppm, Fe$\leq$20 ppm, Cl$\leq$0.001%, SO$_4$$\leq$0.03%, and NO$_3$$\leq$20 ppm per 20 wt% concentration of $Cr_m(A^x)_n$, and is substantially free from chromium (VI).

**EP 2 039 673 A1**

**Description**

Technical Field:

This invention relates to an organic acid chromium (III) salt aqueous solution and a process of producing the same.

Background Art

It is known that chromium (III) oxalate, one of organic acid chromium (III) salts, is prepared by, for example, the following process (See Non-Patent Document 1). An aqueous solution of an inorganic salt of chromium (III), e.g., chromium (III) sulfate, chromium (III) nitrate or chromium (III) chloride, is neutralized by addition of a sodium hydroxide aqueous solution or aqueous ammonia to precipitate chromium hydroxide. The precipitate is dissolved in an oxalic acid solution, which is concentrated to give chromium (III) oxalate. An aqueous solution of the thus obtained chromium (III) oxalate contains metal ions, e.g., Na ions or Fe ions, and anions, e.g., $Cl^-$, $SO_4^{2-}$ or $NO_3^-$, as trace impurities originated in the starting materials. These trace impurities are unavoidable in the production of a chromium (III) oxalate aqueous solution as long as the above-described process is followed.

Apart from an organic acid chromium (III) salt aqueous solution, Applicant of the present invention previously proposed an aqueous solution of an inorganic acid chromium (III) salt, such as chromium (III) nitrate or chromium (III) chloride (See Patent Document 1). The chromium (III) salt aqueous solution is characterized by containing a reduced amount of oxalic acid. A chromium (III) salt aqueous solution with a reduced amount of oxalic acid, when used in a metal surface treatment or a chromating treatment, has an advantage of providing products with excellent luster. Although Patent Document 1 discloses various inorganic acid salts of chromium (III), it gives no mention of organic acid salts of chromium (III).

Non-Patent Document 1: Encyclopaedia Chimica, compact edition, 14th impression, vol. 4, KYORITSU SHUPPAN CO., LTD., p. 636, (Sep. 15, 1972)
Patent Document 1: WO 2005/056478

Disclosure of the Invention

An object of the present invention is to provide a high purity organic acid chromium (III) salt aqueous solution with reduced impurity and a process of producing the same.

The present invention provides an aqueous solution containing an organic acid chromium (III) salt represented by general formula: $Cr_m(A^x)_n$, wherein A represents a residue left after proton removal from an organic acid; x represents a charge of A; and m and n represent integers satisfying equation $3m+xn=0$. The aqueous solution contains the organic acid chromium (III) salt in a concentration of 6% by weight or higher in terms of $Cr_m(A^x)_n$, has impurity ion concentrations of Na≤30 ppm, Fe≤20 ppm, Cl≤0.001%, SO₄≤0.03%, and NO₃≤20 ppm per 20 wt% concentration of $Cr_m(A^x)_n$, and is substantially free from chromium (VI).

The present invention also provides a preferred process of producing the organic acid chromium (III) salt aqueous solution. The process includes mixing a mixed aqueous solution of an organic acid and an organic reducing agent with a chromic acid (VI) aqueous solution to reduce chromium (VI) to chromium (III).

Best Mode for Carrying out the Invention

The present invention will be described based on its preferred embodiments. The organic acid chromium (III) salt aqueous solution of the invention is a solution of an organic acid chromium (III) salt represented by general formula: $Cr_m(A^z)_n$ in water. The term "chromium" as hereinafter referred to means chromium (III) unless otherwise specified. In the general formula above, A represents a residue left after proton removal from an organic acid; x represents a charge (negative charge) of A; and m and n represent integers satisfying equation $3m+xn=0$.

The organic acid in the organic acid chromium salt is represented by $R(COOH)_y$, wherein R is an organic group, a hydrogen atom, a single bond or a double bond, and y is an integer of 1 or greater representing the number of carboxyl groups in the organic acid. "A" in the above general formula is represented by $R(COO^-)_y$. When R is an organic group, the organic group is preferably an aliphatic group having 1 to 10, still preferably 1 to 5, carbon atoms. The aliphatic group may be substituted with other functional group(s), e.g., a hydroxyl group. The aliphatic group may be either saturated or unsaturated. A saturated aliphatic group is preferred, taking into consideration chromium (VI) reducing performance in the preparation of an organic acid chromium salt aqueous solution described *infra*.

The number of carboxyl groups in the organic acid may be one or more than one. In other words, the organic acid may be a monocarboxylic acid or a polycarboxylic acid. The number of carboxyl groups in the organic acid is preferably 1 to 3.

Preferred organic acids are divided into the following groups (a) to (c):

(a) Organic acids having one carboxyl group and in which the moiety other than the carboxyl group is a hydrogen atom or an unsubstituted or hydroxyl-substituted, saturated aliphatic group having 1 to 5, preferably 1 or 2, carbon atoms. Examples are formic acid, acetic acid, glycolic acid, lactic acid, and gluconic acid.

(b) Organic acids having two carboxyl groups and in which the moiety other than the carboxyl groups is a single bond, a double bond or an unsubstituted or hydroxyl-substituted, saturated aliphatic group having 1 or 2 carbon atoms. Examples are oxalic acid, maleic acid, malonic acid, malic acid, tartaric acid, and succinic acid.

(c) Organic acids having three carboxyl groups and in which the moiety other than the carboxyl groups is an unsubstituted or hydroxyl-substituted, saturated aliphatic group having 1 to 3 carbon atoms, such as citric acid.

The concentration of the organic acid chromium salt in the organic acid chromium salt aqueous solution of the invention is 6% by weight or higher, preferably 12% by weight or higher, even more preferably 20% by weight or higher, in terms of $Cr_m(A^x)_n$. The concentration is adjustable as appropriate to the intended use of the organic acid chromium salt aqueous solution. If the concentration of the organic acid chromium salt in the aqueous solution is less than 6% by weight, there will arise problems. For example, when used as a replenisher for a metal surface treating bath or a chromating bath as described later, the solution will fail to maintain the components of the bath at adequate concentrations. While there is no particular upper limit on the organic acid chromium salt concentration in the aqueous solution, too high a concentration can cause precipitation. Moreover, in too high a concentration, the aqueous solution is apt to be difficult to handle due to high viscosity, ultimately becoming tar-like. The upper limit on the concentration should be decided according to the organic acid chromium salt species because the concentration at which precipitation occurs or the solution becomes tar-like varies depending on the species. In the case of chromium oxalate, for example, the upper limit is preferably 50% by weight, more preferably 40% by weight.

The organic acid chromium salt aqueous solution of the invention is characterized by being substantially free from chromium (VI). Substantial absence of chromium (VI) means high safety of the organic acid chromium salt aqueous solution of the invention. The phrase "substantially free from (or substantial absence of) chromium (VI)" as used herein means that the chromium (VI) concentration in the organic acid chromium salt aqueous solution is lower than the detection limit of an available measuring instrument. The chromium (VI) concentration in the organic acid chromium salt aqueous solution of the invention is measured, e.g., by organic solvent extraction combined with absorption spectrophotometry. The chromium (III) concentration in the solution of the invention is measured, e.g., by ICP-AES.

The organic acid chromium salt aqueous solution of the invention is also characterized by having extremely low concentrations of various impurity ions. Specifically, it has extremely low concentrations of metal ions, e.g., Na and Fe, and anions, e.g., Cl, $SO_4$, and $NO_3$. As to metal ions, Na$\leq$30 ppm and Fe$\leq$20 ppm, preferably Fe$\leq$10 ppm, each per 20 wt% concentration of $Cr_m(A^x)_n$. As for anions, Cl$\leq$0.001%, SO$\leq$0.03%, preferably $SO_4\leq$0.02%, and $NO_3\leq$20 ppm per 20 wt% concentration of $Cr_m(A^x)_n$. These impurity ions are considered to have adverse influences on the finish when the organic acid chromium salt aqueous solution is used in, for example, metal surface treatment or chromating treatment. Accordingly, application of the organic acid chromium salt aqueous solution with extremely low concentrations of these impurity ions to such uses is expected to give a good finish. The impurity ion concentrations of the organic acid chromium salt aqueous solution can be measured by, for example, ICP-AES. In the description of the invention, all the percents and ppms are by weight unless otherwise noted.

It is preferred that the organic acid chromium salt aqueous solution of the invention be substantially free from a free organic acid. In application to, for example, metal surface treatment or chromating treatment, a free organic acid in the organic acid chromium salt aqueous solution can adversely affect the finish. The phrase "substantially free from a free organic acid" means that the concentrations of chromium and an organic acid in the aqueous solution satisfy the stoichiometry represented by formula $Cr_m(A^x)_n$ within the range of measurement error.

It is preferred that the organic acid chromium salt aqueous solution of the invention be substantially free from a half-oxidized product of the organic acid. In application to, for example, a metal surface treatment or a chromating treatment, a half-oxidized product of the organic acid in the organic acid chromium salt aqueous solution can adversely affect the finish. As will be apparent from the process of producing the organic acid chromium salt aqueous solution described later, the organic acid not only supplies a counter-ion to a chromium (III) ion but also serves as a reducing agent for chromium (VI). Therefore, the organic acid is oxidized ultimately into water and carbon dioxide. Under some conditions of chromium (VI) reduction, cases are met with in which oxidation reaction of the organic acid stops halfway. Should this occur, there will be a half-oxidized product of the organic acid in the aqueous solution. The phrase "substantially free from a half-oxidized product of an organic acid" means that the concentration of the half-oxidized product is lower than the detection limit in analyzing ions present in the organic acid chromium salt aqueous solution by, e.g., ion chromatography.

The organic acid chromium salt aqueous solution according to the present invention is suited for use as a chromium plating bath in the surface treatment of various metals. For example, it is used in decorative final finishing or in plating a nickel-plated surface. It is also suited for use in chromating a zinc- or tin-plated surface. The organic acid chromium salt aqueous solution of the invention is particularly useful as a replenisher for a chromium plating bath for metal surface treatment or as a replenisher for a chromating bath. The composition of the chromium plating or chromating bath is liable to change due to difference between anions in introduceability into the coating film. Compared with organic anions that form complexes with chromium (III) easily, inorganic anions such as sulfate, nitrate and chloride ions are less introduceable into the film and therefore more accumulated in the bath. In cases where the inorganic anion concentration in the bath is low with respect to chromium (III), the composition of the bath can be regulated relatively easily by adding an inorganic chromium salt, such as chromium sulfate, chromium nitrate or chromium chloride, as a chromium source. In cases where the inorganic anion concentration becomes higher than necessary, compositional regulation is difficult. In contrast, because organic anions, which are easily introduceable into the film in the form of chromium (III) complexes, are hardly accumulated in the bath, addition of the organic acid chromium salt aqueous solution of the invention to the bath as a chromium source results in little change in bath composition. Therefore, the bath can serve for a prolonged period of time, thus eliminating the need of frequent replacement.

The organic acid chromium salt aqueous solution of the invention is also useful as a catalyst or a starting material for producing dielectric substances such as barium titanate. In the production of dielectric substances such as barium titanate, chromium is added in some cases as a trace component to improve the performance. Using the organic acid chromium salt aqueous solution of the invention as the chromium source offers an advantage that the organic matter is removed from a dielectric substance during firing, thereby to give a product with reduced impurity.

A preferred process of producing the organic acid chromium salt aqueous solution of the invention will then be described. The process according to the present invention includes mixing a mixed aqueous solution of an organic acid and an organic reducing agent with a chromic (VI) acid aqueous solution to reduce chromium (VI) to chromium (III).

The chromic (VI) acid aqueous solution as a starting material is prepared by, for example, dissolving chromium trioxide (chromium (VI) oxide) in water. Chromium trioxide can be obtained from sodium chromate through various purification treatments. Sodium chromate is obtainable by oxidative roasting of chrome ores under alkaline conditions. The thus prepared chromic (VI) acid aqueous solution has extremely small contents of impurities such as Fe, Na, Mg, Al, Ca, Ni, Mo, and W, as compared with a chromic acid aqueous solution prepared from chromium hydroxide obtained by adding caustic soda or soda ash to chromium sulfate or from chromium carbonate or a chromic acid aqueous solution prepared by dissolving high-carbon ferrochrome in sulfuric acid or hydrochloric acid.

It is only necessary that the chromic (VI) acid aqueous solution be a solution in the reaction system. It is possible to use chromium trioxide at the beginning of the reaction. In most cases, nevertheless, an aqueous solution previously prepared by adding water to chromium trioxide to dissolve is used. The concentration of the chromic (VI) acid aqueous solution is not particularly limited but generally ranges from 15% to 60% by weight.

The organic acid is chosen from those recited *supra*. Any organic reducing agent can be used as long as it almost decomposes into carbonic acid gas and water in a reduction reaction hereinafter described, leaving no substantial organic decomposition products. Examples of such an organic reducing agent include monohydric alcohols, e.g., methyl alcohol and propyl alcohol; dihydric alcohols, e.g., ethylene glycol and propylene glycol; monosaccharides, e.g., glucose; disaccharides, e.g., maltose; and polysaccharides, e.g., starch.

Some organic acids can act as an organic reducing agent in the process of the invention. In such a case, there is no need to use an organic reducing agent separately. Using no organic reducing agent separately simplifies the process and reduces the cost. Examples of organic acids serving as an organic reducing agent include monocarboxylic acids, e.g., lactic acid, gluconic acid, and glycolic acid; dicarboxylic acids, e.g., oxalic acid, malic acid, maleic acid, malonic acid, and tartaric acid; and tricarboxylic acids, e.g., citric acid.

In cases where an organic reducing agent is used in addition to the organic acid, it is used in an amount required to reduce chromium (VI) to chromium (III). It is preferred that the organic reducing agent and the organic acid be mixed and used in the form of a mixed aqueous solution. In using an organic acid acting as an organic reducing agent, it is used in an amount equal to the sum of the amount required to reduce chromium (VI) and the amount required to produce an organic acid chromium (III) salt. Taking oxalic acid, for instance, reduction of chromium (VI) and production of chromium (III) oxalate proceed as represented by reaction formula:

$$6(COOH)_2 + 2CrO_3 \rightarrow Cr_2(C_2O_4)_3 + 6CO_2 + 6H_2O$$

Taking the theoretical amount of oxalic acid required to convert chromic acid to chromium oxalate as "a", and that required to reduce chromic acid as "b", the a to b molar ratio is basically 1:1 as shown by the above reaction formula. So the amount of oxalic acid to be added is the sum of the amount required to produce chromium oxalate and that required to reduce chromic (VI) acid.

In using an organic acid acting as an organic reducing agent, an aqueous solution of the organic acid may be added to the chromic (VI) acid aqueous solution, or the latter may be added to the former. When the organic acid has low water solubility, for example, when in using oxalic acid, it is advisable that the organic acid be dissolved in water by heating in a reaction vessel to prepare an aqueous solution with an increased concentration, into which the chromic (VI) acid aqueous solution be added, whereby a high concentration organic acid chromium salt aqueous solution can be obtained. When in using an organic acid with high water solubility, needing no heating for dissolving, it is dissolved in water at room temperature, and the resulting aqueous solution is added to the chromic (VI) acid aqueous solution. Addition of an aqueous solution of a certain kind of an organic acid to the chromic acid aqueous solution can cause the reaction system to gel. If such is the case, the chromic acid aqueous solution should be added to the organic acid aqueous solution.

Reduction of chromium (VI) occurs upon mixing chromic (VI) acid, the organic acid, and, if necessary, the organic reducing agent. Because this reaction is a redox reaction accompanied by vigorous heat generation, the reaction solution temperature rapidly rises up to the boiling point. The reaction temperature is usually between 90° and 110°C. After completion of the reaction, the reaction system is aged for more than 30 minutes, preferably more than 1 hour. The aging temperature is not particularly limited and may be the temperature at the end of the reaction. The carbon dioxide generated by the oxidation of the organic acid or the organic reducing agent is released out of the reaction system. Completion of the reaction is evidenced by the absence of chromium (VI) in the reaction solution, i.e., by confirming that chromium (VI) is below the detection limit. After the aging, the organic acid may be added if the chromium (III) to organic acid molar ratio needs to be adjusted.

All the water evaporated by the heat generated by the reduction of chromium (VI) may be returned as reflux to the reaction system. For the purpose of increasing the concentration of the organic acid chromium salt, part of the water may be withdrawn from the reaction system, with the remainder being returned to the reaction system as reflux. By this operation, a high concentration organic acid chromium salt solution can be obtained directly without providing a separate step of concentration. The organic acid chromium salt concentration of the resulting solution will be as high as 20% by weight or more, preferably 25% by weight or more.

While the invention has been described with reference to its preferred embodiments, it should be understood that the invention is not deemed to be limited thereto, and various modifications based on the disclosure will occur to those skilled in the art. All such modifications within the scope of the appended claims are included in the invention.

Examples

The present invention will now be illustrated in greater detail by way of Examples. Unless otherwise noted, all the percents are by weight.

Example 1

In a 1-liter glass reaction vessel equipped with a reflux condenser was put 245.5 g of water, and 312.5 g of oxalic acid dihydrate was poured therein while stirring. The reaction vessel was heated up to 80°C to completely dissolve the oxalic acid dihydrate under reflux to prepare a 40% oxalic acid aqueous solution. The amount of oxalic acid present in the solution was the sum of the amount required to produce chromium oxalate and the amount required to reduce chromic (VI) acid.

To the oxalic acid aqueous solution was added 551.1 g of a 15% chromic acid aqueous solution (corresponding to the equivalent) at a rate of 3.0 ml/min over a period of about 3 hours to conduct reduction of chromium (VI). Five minutes from the start of addition, the reaction temperature rose to about 100°C. Part of the evaporated water generated was withdrawn from the reaction system, and the remainder was returned to the reaction system as reflux. The water withdrawn weighed about 250 g. Carbon dioxide generated was released out of the system. After completion of the chromic acid addition, the reaction solution was aged for more than 30 minutes. Chromium (VI) in the solution was analyzed, and when found to be below the detection limit, it was taken as the end point of the reaction. Chromium (VI) was detected by organic solvent extraction coupled with absorption spectrophotometry. The resulting chromium oxalate aqueous solution was assayed to determine chromium concentration and oxalic acid concentration. As a result, formation of $Cr_2(C_2O_4)_3$ was confirmed. The results of the assay are shown in Table 1.

TABLE 1

|  | Concentration |
|---|---|
| $Cr_2(C_2O_4)_3$ (%) | 20.3 |
| Cr (VI) (ppm) | undetected |
| Na (ppm) | 19 |
| Fe (ppm) | 4 |
| Ca (ppm) | 3 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 2

A chromium oxalate aqueous solution was prepared in the same manner as in Example 1, except that all the water evaporated was returned as reflux to the reaction system. The resulting chromium oxalate aqueous solution was assayed for chromium concentration and oxalic acid concentration to confirm the formation of $Cr_2(C_2O_4)_3$. The results of the assay are shown in Table 2 below.

TABLE 2

|  | Concentration |
|---|---|
| $Cr_2(C_2O_4)_3$ (%) | 15.2 |
| Cr (VI) (ppm) | undetected |
| Na (ppm) | 15 |
| Fe(ppm) | 3 |
| Ca (ppm) | 3 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 3

In a 1-liter glass reaction vessel equipped with a reflux condenser were put 151.4 g of a 60% chromic acid aqueous solution and 302.9 g of water, resulting in a chromic acid concentration of 20%. Separately, 180.8 g of malonic acid was dissolved in 409.8 g of water to prepare a malonic acid aqueous solution having a concentration of 30%. The amount of malonic acid present in the resulting solution was the sum of the amount required to produce chromium malonate and the amount required to reduce chromic (VI) acid.

The malonic acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it, which weighed about 260 g. Carbon dioxide was released out of the system. After completion of addition of the malonic acid aqueous solution, the reaction solution was aged for more than 30 minutes. Thereafter, the reaction solution was treated in the same manner as in Example 1 to obtain a chromium malonate aqueous solution. The resulting chromium malonate aqueous solution was assayed for chromium and malonic acid concentrations to confirm the formation of $Cr_2(C_3H_2O_4)_3$. The results of the assays are shown in Table 3.

TABLE 3

|  | Concentration |
| --- | --- |
| $Cr_2(C_3H_2O_4)_3$ (%) | 25.2 |
| Cr (VI) (ppm) | undetected |
| Na (ppm) | 18 |
| Fe (ppm) | 5 |
| Ca (ppm) | 2 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 4

In a 1-liter glass reaction vessel equipped with a reflux condenser were put 147.1 g of a 60% chromic acid aqueous solution and 294.3 g of water, resulting in a chromic acid concentration of 20%. Separately, 181.0 g of maleic acid was dissolved in 416.4 g of water to prepare a maleic acid aqueous solution having a concentration of 30%. The amount of maleic acid present in the resulting solution was the sum of the amount required to produce chromium maleate and the amount required to reduce chromic (VI) acid.

The maleic acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the reaction system, which weighed about 210 g. Carbon dioxide was released out of the system. After completion of addition of the maleic acid aqueous solution, the reaction solution was aged for more than 30 minutes. Thereafter, the reaction solution was treated in the same manner as in Example 1 to obtain a chromium maleate aqueous solution. The resulting chromium maleate aqueous solution was assayed for chromium and maleic acid concentrations to confirm the formation of $Cr_2(C_4H_2O_4)_3$. The results of the assays are shown in Table 4.

TABLE 4

|  | Concentration |
| --- | --- |
| $Cr_2(C_4H_2O_4)_3$ (%) | 25.0 |
| Cr (VI) (ppm) | undetected |
| Na (ppm) | 18 |
| Fe (ppm) | 4 |

(continued)

|  | Concentration |
|---|---|
| Ca (ppm) | 2 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 5

In a 1-liter glass reaction vessel equipped with a reflux condenser were put 134.7 g of a 60% chromic acid aqueous solution and 269.3 g of water, resulting in a chromic acid concentration of 20%. Separately, 191.4 g of malic acid was dissolved in 440.2 g of water to prepare a malic acid aqueous solution having a concentration of 30%. The amount of malic acid present in the resulting solution was the sum of the amount required to produce chromium malate and the amount required to reduce chromic (VI) acid.

The malic acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the reaction system, which weighed about 190 g. Carbon dioxide was released out of the system. After completion of addition of the malic acid aqueous solution, the reaction solution was aged for more than 30 minutes. Thereafter, the reaction solution was treated in the same manner as in Example 1 to obtain a chromium malate aqueous solution. The resulting chromium malate aqueous solution was assayed for chromium and malic acid concentrations to confirm the formation of $Cr_2$ $(C_4H_4O_5)_3$. The results of the assays are shown in Table 5.

TABLE 5

|  | Concentration |
|---|---|
| $Cr_2(C_4H_4O_5)_3$ (%) | 25.1 |
| Cr (VI) (ppm) | undetected |
| Na (ppm) | 16 |
| Fe (ppm) | 4 |
| Ca (ppm) | 3 |
| Mg (ppm) | undetected |
| Al (ppm) | undetected |
| Cu (ppm) | undetected |
| Ni ppm) | undetected |
| Cl (%) | <0.001 |
| $SO_4$ (%) | 0.01 |
| $NO_3$ (ppm) | <20 |

Example 6

In a 1-liter glass reaction vessel equipped with a reflux condenser were put 138.6 g of a 60% chromic acid aqueous solution and 277.2 g of water, resulting in a chromic acid concentration of 20%. Separately, 204.7 g of citric acid was dissolved in 416.1 g of water to prepare a citric acid aqueous solution having a concentration of 30%. The amount of citric acid present in the resulting solution was the sum of the amount required to produce chromium citrate and the amount required to reduce chromic (VI) acid.

The citric acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the system, which weighed about 200 g. Carbon dioxide was released out of the system. After completion of addition of the citric acid aqueous solution, the reaction solution was aged for more than 30 minutes. Thereafter, the reaction solution was treated in the same manner as in Example 1 to obtain a chromium citrate aqueous solution. The resulting chromium citrate aqueous solution was assayed for chromium and citric acid concentrations to confirm the formation of $Cr(C_6H_5O_7)$. The results of the assays are shown in Table 6.

TABLE 6

|  | Concentration |
|---|---|
| $Cr(C_6H_5O_7)$ (%) | 25.2 |
| Cr (VI) (ppm) | undetected |
| Na (ppm) | 19 |
| Fe (ppm) | 6 |
| Ca (ppm) | 4 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 7

In a 1-liter glass reaction vessel equipped with a reflux condenser were put 115.6 g of a 60% chromic acid aqueous solution and 231.2 g of water, resulting in a chromic acid concentration of 20%. Separately, 227.9 g of 90% lactic acid was dissolved in 448.2 g of water to prepare a lactic acid aqueous solution having a concentration of 30%. The amount of lactic acid present in the resulting solution was the sum of the amount required to produce chromium lactate and the amount required to reduce chromic (VI) acid.

The lactic acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the system, which weighed about 260 g. Carbon dioxide was released out of the system. After completion of addition of the lactic acid aqueous solution, the reaction solution was aged for more than 30 minutes. Thereafter, the reaction solution was treated in the same manner as in Example 1 to obtain a chromium lactate aqueous solution. The resulting chromium lactate aqueous solution was assayed for chromium and lactic acid concentrations to confirm the formation of $Cr(C_3H_5O_3)_3$. The results of the assays are shown in Table 7.

TABLE 7

|  | Concentration |
|---|---|
| $Cr(C_3H_5O_3)_3$ (%) | 30.1 |
| Cr (VI) (ppm) | undetected |

(continued)

|  | Concentration |
|---|---|
| Na (ppm) | 14 |
| Fe (ppm) | 5 |
| Ca (ppm) | 3 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 8

In a 1-litter glass reaction vessel equipped with a reflux condenser were put 124.1 g of a 60% chromic acid aqueous solution and 248.3 g of water, resulting in a chromic acid concentration of 20%. Separately, 283.0 g of 70% glycolic acid was dissolved in 377.4 g of water to prepare a glycolic acid aqueous solution having a concentration of 30%. The amount of glycolic acid present in the resulting solution was the sum of the amount required to produce chromium glycolate and the amount required to reduce chromic (VI) acid.

The glycolic acid aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the system, which weighed about 200 g. Carbon dioxide was released out of the system. After completion of addition of the glycolic acid aqueous solution, the reaction solution was aged for more than 30 minutes. Thereafter, the reaction solution was treated in the same manner as in Example 1 to obtain a chromium glycolate aqueous solution. The resulting chromium glycolate aqueous solution was assayed for chromium and glycolic acid concentrations to confirm the formation of $Cr(C_2H_3O_3)_3$. The results of the assays are shown in Table 8.

TABLE 8

|  | Concentration |
|---|---|
| $Cr(C_2H_3O_3)_3$ (%) | 25.6 |
| Cr (VI) (ppm) | undetected |
| Na (ppm) | 17 |
| Fe (ppm) | 4 |
| Ca (ppm) | 2 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 9

In a 1-liter glass reaction vessel equipped with a reflux condenser was put 661.0 g of 50% gluconic acid. The amount of gluconic acid present in the aqueous solution was the sum of the amount required to produce chromium gluconate and the amount required to reduce chromic (VI) acid. Separately, 89.6 g of a 60% chromic acid aqueous solution was dissolved in 268.8 g of water to prepare a chromic acid aqueous solution having a concentration of 15%.
The chromic (VI) acid aqueous solution was added to the gluconic acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the system, which weighed about 150 g. Carbon dioxide was released out of the system. After completion of addition of the chromic (VI) acid aqueous solution, the reaction solution was aged for more than 30 minutes. Thereafter, the reaction solution was treated in the same manner as in Example 1 to obtain a chromium gluconate aqueous solution. The resulting chromium gluconate aqueous solution was assayed for chromium and gluconic acid concentrations to confirm the formation of Cr $(C_6H_{11}O_7)_3$. The results of the assays are shown in Table 9.
TABLE 9

|  | Concentration |
|---|---|
| $Cr(C_6H_{11}O_7)_3$ (%) | 40.2 |
| Cr (VI) (ppm) | undetected |
| Na (%) | 12 |
| Fe (ppm) | 2 |
| Ca (ppm) | 2 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 10

In a 1-liter glass reaction vessel equipped with a reflux condenser were put 211.6 g of tartaric acid and 312.1 g of water to prepare a 40% tartaric acid aqueous solution. The amount of tartaric acid present in the aqueous solution was the sum of the amount required to produce chromium tartrate and the amount required to reduce chromic (VI) acid. Separately, 129.3 g of a 60% chromic acid aqueous solution was dissolved in 387.9 g of water to prepare a 15% chromic acid aqueous solution.
The chromic (VI) acid aqueous solution was added to the tartaric acid aqueous solution at a rate of about 5 ml/min to conduct reduction of chromium (VI). Thirty minutes from the start of addition, the reaction temperature rose to about 90°C. The evaporated water generated was returned as reflux while withdrawing part of it from the system, which weighed about 160 g. Carbon dioxide was released out of the system. After completion of addition of the chromic (VI) acid aqueous solution, the reaction solution was aged for more than 30 minutes. Thereafter, the reaction solution was treated in the same manner as in Example 1 to obtain a chromium tartrate aqueous solution. The resulting chromium tartrate aqueous solution was assayed for chromium and tartaric acid concentrations to confirm the formation of $Cr_2$ $(C_4H_4O_6)_3$. The results of the assays are shown in Table 10.
TABLE 10

| | Concentration |
|---|---|
| $Cr_2(C_4H_4O_6)_3$ (%) | 25.4 |
| Cr (VI) (ppm) | undetected |
| Na (ppm) | 16 |
| Fe (ppm) | 6 |
| Ca (ppm) | 4 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.01 |
| $NO_3$ (ppm) | <20 |

Example 11

In a 1-liter glass reaction vessel equipped with a reflux condenser were put 300.6 g of a 60% chromic acid aqueous solution and 150.3 g of water. The resultant chromic acid concentration in the reaction vessel was 40%. Separately, 405.8 g of 80% acetic acid and 41.8 g of glucose as an organic reducing agent were dissolved in 161.1 g of water to prepare a mixed aqueous solution having acetic acid and glucose concentrations of 53% and 6.7%, respectively.

The mixed aqueous solution was added to the chromic (VI) acid aqueous solution at a rate of about 5 ml/min to conduct reduction of chromium (VI). Thirty minutes from the start of addition, the reaction temperature rose to about 90°C. The evaporated water generated was returned to the system as reflux, and carbon dioxide was released out of the system. After completion of addition of the mixed aqueous solution, the reaction solution was aged for more than 30 minutes. Chromium (VI) in the reaction solution was analyzed, a glucose aqueous solution was added, and the aging was continued. When chromium (VI) was below the detection limit, it was regarded as the reaction end point. The resulting chromium acetate aqueous solution was assayed for chromium and acetic acid concentrations to confirm the formation of $Cr(C_2H_3O_2)_3$. The results of the assays are shown in Table 11.

TABLE 11

| | Concentration |
|---|---|
| $Cr(C_2H_3O_2)_3$ (%) | 40.1 |
| Cr (VI) (ppm) | undetected |
| Na (ppm) | 26 |
| Fe (ppm) | 6 |
| Ca (ppm) | 4 |
| Mg (ppm) | undetected |
| Al (ppm) | undetected |
| Cu (ppm) | undetected |
| Ni (ppm) | undetected |
| Cl (%) | <0.001 |
| $SO_4$ (%) | 0.02 |
| $NO_3$ (ppm) | <20 |

Comparative Example 1

In a 1-liter glass reaction vessel equipped with a reflux condenser were put 207 g of a 60% chromic acid aqueous solution and 103.5 g of water. In the reaction vessel was put 186.3 g of 98% sulfuric acid, followed by stirring well. An aqueous solution of 28.8 g of 97% glucose in 119.4 g of water was put into the reaction vessel over a period of 2 hours to conduct reduction reaction. The solution temperature at the end of the reaction was about 110°C. There was obtained 604 g of a chromium sulfate aqueous solution having a $Cr_2(SO_4)_3$ concentration of 40%. To the resulting chromium sulfate aqueous solution was added 745 g of 20% sodium hydroxide over a period of 1 hour, whereupon chromium hydroxide precipitated. The slurry containing chromium hydroxide was filtered on a 12.5 cm Buechner funnel to give about 700 g of chromium hydroxide, which was found to contain a large amount of sodium sulfate. The filter cake as obtained was re-slurried and filtered three times each using 10 times as much water as the cake to give 580 g of chromium hydroxide. The results of analysis on the product are shown in Table 12 below.
TABLE 12

|  | Concentration |
|---|---|
| $Cr(OH)_3$ (%) | 22.0 |
| Water content (%) | 78.0 |
| Na (ppm) | 210 |
| $SO_4$ (%) | 0.80 |

A 500 g portion of the water-containing chromium hydroxide as obtained was added to 505 g of a 40% oxalic acid aqueous solution heated to 80°C to effect dissolution reaction to give a chromium oxalate aqueous solution. The resulting chromium oxalate aqueous solution was analyzed for chromium oxalate concentration and by-produced impurity contents. The results obtained are shown below.
TABLE 13

|  | Concentration |
|---|---|
| $Cr_2(C_2O_4)_3$ (%) | 19.6 |
| Cr (VI) (ppm) | undetected |
| Na (ppm) | 108 |
| $SO_4$ (%) | 0.41 |

Industrial Applicability

As described, the present invention has achieved considerable decrease in the amount of various impurity ions that are unavoidably present in an organic acid chromium (III) salt aqueous solution conventionally produced on an industrial scale.

**Claims**

1. An aqueous solution containing an organic acid chromium (III) salt represented by general formula: $Cr_m(A^x)_n$, wherein A represents a residue left after proton removal from an organic acid; x represents a charge of A; and m and n represent integers satisfying equation $3m+xn=0$, wherein:
the aqueous solution contains the organic acid chromium (III) salt in a concentration of 6% by weight or higher in terms of $Cr_m(A^x)_n$, has impurity ion concentrations of Na≤30 ppm, Fe≤20 ppm, Cl≤0.001%, $SO_4$≤0.03%, and $NO_3$≤20 ppm per 20 wt% concentration of $Cr_m(A^x)_n$, and is substantially free from chromium (VI).

2. The aqueous solution according to claim 1, wherein the organic acid chromium (III) salt is chromium (III) oxalate, chromium (III) lactate, chromium (III) citrate, chromium (III) malate, chromium (III) gluconate, chromium (III) maleate, chromium (III) malonate, chromium (III) tartrate, chromium (III) glycolate or chromium (III) acetate.

3.  The aqueous solution according to claim 1 or 2, which is substantially free from the organic acid in its free form.

4.  The aqueous solution according to claim 1, which is substantially free from a half-oxidized product of the organic acid.

5.  The aqueous solution according to claim 1, which is used as a replenisher for a metal surface treatment or chromating treatment bath.

6.  A process of producing the aqueous solution containing the organic acid chromium (III) salt according to claim 1, which comprises mixing a mixed aqueous solution of an organic acid and an organic reducing agent with a chromic (VI) acid aqueous solution to reduce chromium (VI) to chromium (III).

7.  The process according to claim 6, wherein the organic acid serves as the organic reducing agent and is used in an amount equal to the sum of an amount required to produce the organic acid chromium (III) salt and an amount required to reduce chromium (VI), and no organic reducing agent other than the organic acid is used.

8.  The process according to claim 7, wherein an aqueous solution of the organic acid is added to the chromic (VI) acid aqueous solution.

9.  The process according to claim 7, wherein the chromic (VI) acid aqueous solution is added to an aqueous solution of the organic acid.

10. The process according to claim 6, wherein part of water evaporated by the heat generated by the reduction of chromium (VI) is withdrawn from the reaction system, with the remainder being returned as reflux to the reaction system, to concentrate the reaction system.

## INTERNATIONAL SEARCH REPORT

| | International application No. |
|---|---|
| | PCT/JP2007/060107 |

A.  CLASSIFICATION OF SUBJECT MATTER
$C07C51/41$(2006.01)i, $C07C53/10$(2006.01)i, $C07C55/06$(2006.01)i, $C07C55/08$
(2006.01)i, $C07C55/24$(2006.01)i, $C07C59/06$(2006.01)i, $C07C59/08$(2006.01)i,
$C07C59/105$(2006.01)i, $C07C59/245$(2006.01)i, $C07C59/255$(2006.01)i,
According to International Patent Classification (IPC) or to both national classification and IPC

B.  FIELDS SEARCHED

Minimum documentation searched (classification system followed by classification symbols)
$C07C51/41$, $C07C53/00-59/92$

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)
CA(STN), REGISTRY(STN)

C.  DOCUMENTS CONSIDERED TO BE RELEVANT

| Category* | Citation of document, with indication, where appropriate, of the relevant passages | Relevant to claim No. |
|---|---|---|
| X<br>A | JP 2002-523391 A  (Beijing Juneng Asia Pacific<br>Life Scientific Research Center),<br>30 July, 2002 (30.07.02),<br>Claims; examples<br>& WO 2000/010962 A1     & EP 1106599 A1<br>& US 6548687 B1 | 1,3-4<br>2,5-10 |
| A | JP 2004-520265 A  (Societe Nouvelle des<br>Couleurs Zinciques),<br>08 July, 2004 (08.07.04),<br>Claims<br>& WO 2002/010179 A1     & FR 2812640 A1<br>& AU 200178564 A       & EP 1305325 A1<br>& CN 1444591 A | 1-10 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 June, 2007 (08.06.07) | 26 June, 2007 (26.06.07) |

| Name and mailing address of the ISA/<br>Japanese Patent Office | Authorized officer |
|---|---|
| Facsimile No. | Telephone No. |

Form PCT/ISA/210 (second sheet) (April 2005)

**INTERNATIONAL SEARCH REPORT**

International application No.

PCT/JP2007/060107

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-194167 A (The Nippon Chemical Industrial Co., Ltd.), 21 July, 2005 (21.07.05), Claims & WO 2005/056478 A1  & JP 2005-314724 A & JP 2005-325384 A  & EP 1712524 A1 | 1-10 |

Form PCT/ISA/210 (continuation of second sheet) (April 2005)

16

**INTERNATIONAL SEARCH REPORT**

International application No.

PCT/JP2007/060107

Continuation of A. CLASSIFICATION OF SUBJECT MATTER
   (International Patent Classification (IPC))

*C07C59/265*(2006.01)i



         (According to International Patent Classification (IPC) or to both national
         classification and IPC)


Continuation of B. FIELDS SEARCHED
   Minimum documentation searched (International Patent Classification (IPC))




         Minimum documentation searched (classification system followed by
         classification symbols)

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005056478 A

**Non-patent literature cited in the description**

- Encyclopaedia Chimica. KYORITSU SHUPPAN CO., LTD, 15 September 1972, vol. 4, 636